# EUROPEAN PATENT APPLICATION

(11) **EP 2 047 831 A1**
(43) Date of publication of application: **15.04.2009**
(21) Application number: 07768458.7
(22) Date of filing: 23.07.2007
(51) Int. Cl.: A61G 7/05, A47C 27/10, A61H 39/04, A61N 1/44

(54) **BED FOR PREVENTING BED SORES**

(30) Priority: 03.08.2006 JP 2006212339
(71) Applicant: BHPH Company Limited, Nassau (BS)
(72) Inventor: HATA, Tadayo, Osaka-shi Osaka 545-0053 (JP)
(74) Representative: Vossius & Partner
(86) International application number: PCT/JP2007/064434
(87) International publication number: WO 2008/015928

(57) **Abstract**

An anti-bedsore bed, comprising multiple expandable/contractable air bags 1 placed in parallel with each other in the width direction of the bed, an air-supplying/discharging means 2 of expanding and contracting the air bags 1 by supplying and discharging compressed air into and out of the air bags 1 at a particular interval t, and a control means 3 of controlling the air-supplying/discharging means 2, wherein the air bags 1 are formed cylindrical in the expanded state in such a manner that possible bedsore-developing regions of a care-receiver A are elevated and lowered at a particular interval t, and the air bags 1 has a blood circulation-accelerating unit 4 made of a material accelerating blood circulation of the care-receiver A. The blood circulation-accelerating unit 4 removes muscle stiffness by accelerating blood circulation and prevents bedsore of the care receiver who are in the bedridden state.

## Description

### TECHNICAL FIELD

The present invention relates to an anti-bedsore bed, more specifically to an anti-bedsore bed for care-receivers such as patients bedridden by disease and bedridden elderly.

### BACKGROUND ART

Generally, healthy people change their postures several times during sleep. For example, assuming that healthy people have normally a sleeping period of 8 hours, they change their postures about over approximately 60 times during the period. The change in posture is physiological in nature and called physical movement. In principle in the body movement of an animal human, "it can keep the same posture for a period of up about 15 minutes". Care-receivers such as patients bedridden by sever injury or disease and bedridden elderly cannot exhibit this body movement and remains in the same posture. If excessive load is applied to the shoulder or waist of the care-receiver, it leads to narrowing of the blood vessels in the muscle of the region and thus deterioration in blood circulation there, which in turn leads to insufficient supply of oxygen and nutrients into the muscle. As a result, the waste products generated remain in the muscle, causing stiffness in the care-receiver.
In addition, care-receivers in the bedridden state, if left in the same posture for a long period, suffer local dysdiemorrhysis, i.e., bedsore, which in turn induces infectious diseases. It can shorten the lives especially of elderly incontinent care-receivers.

Currently in Japan, the most-aged society in the world, where there are more than 4,000,000 elderly eligible for nursing-care insurance, there are only limited absolute numbers of physicians, nurses and well-trained care-givers in the setting of various institutions for the 4000,000 aged care-receivers.

No suitable nursing-care system was enforced in the confusion caused by discrepancy between the administrative intention and miserable medical settings or common sense, and there is practically no advancement in medical education in this field.
More specifically, administrative authorities established a nursing-care system, based on the old medical concept in the 18th century that patients with disease should stay in bed, especially lying quietly in bed after surgery". A nursing-care system far from desired one was implemented and proven to be unsuccessful without any practically favorable results obtained, in the confusion caused by the discrepancy between the administrative intention and practical medical settings.

As described above, there is worsening insufficiency in the quality and quantity of the care-givers in various institutions for elderly. There have been many kinds of beds for care-receivers in the bedridden state proposed for alleviation of the load applied to the care-givers (see e.g., Patent Document 1).
Patent Document 1 discloses a nursing-care bed comprising a bed main body having a mattress unit having a reclining unit, a sitting unit, a leg-receiving unit and a foot-resting unit that are rotatably connected to each other that can be deformed into a chair or bed shape, wherein the bed comprises an additional elevating/lowering apparatus for elevating and lowering the bed main body with the mattress material. In Patent Document 1, the labor by a care-giver needed to move a care-receiver onto a patient-carrying bed or a wheelchair is reduced, as the bed is made convertible from the bed shape to the chair shape by the elevating/lowering apparatus.
Patent Document 1: JP-A No. 2004-16370

### Disclosure of the Invention

### Problems to be Solved by the invention

However, conventional nursing-care beds such as that disclosed in the Patent Document 1 did not have functions to reduce muscle stiffness and prevent bedsore of a care-receiver, although they are convertible between the chair and bed shapes.
In addition, the conventional nursing-care beds, in which the elevating/lowering apparatus was configured with a motor and a link mechanism, were complicated in the structure of the bed elevating/lowering apparatus.
Thus, an object of the present invention is to provide a bed that removes muscle stiffness by accelerating blood circulation and prevents bedsore of care-receivers who are in the bedridden state, as left behind because of labor shortage. Another object of the present invention is to provide a bed allowing facile exchange of care-receiver's diaper.

### Means to Solve the Problems

The anti-bedsore bed according to the present invention, which achieved the objects above, is an anti-bedsore bed, comprising multiple expandable/contractable air bags placed in parallel with each other in the width direction of the bed, an air-supplying/discharging means of expanding and contracting the air bags by supplying and discharging compressed air into and out of the air bags at a particular interval t, and a control means of controlling the air-supplying/discharging means, wherein the air bags are formed cylindrical in the expanded state in such a manner that possible bedsore-developing regions of the care-receiver are elevated and lowered at a particular interval t, and each of the air bags has a blood circulation-accelerating unit made of a material accelerating blood circulation of the care-receiver.
The blood circulation-accelerating unit is preferably formed by molding a blend of a powder of magnet, tourmaline, germanium, Togoal Warmtite, quartz diorite porphyrite, granite porphyry, Hornblende-cummingtonite, Chinese silica, silicon compound, lead-containing barite, or Bincho charcoal with a synthetic resin or rubber.
Each air bag above preferably has a bag main body cylindrical in the expanded state and a flexible sheet-shaped blood circulation-accelerating unit formed on top of the bag main body.
The air bags are preferably arranged at positions corresponding to at least two positions of sural region, femoral region, lumbar region, back region, and suboccipital region of the care-receiver in the supine position. The air bags are more preferably arranged at the positions corresponding to the sural region, femoral region, lumbar region, back region, and suboccipital region.

### Effect of the Invention

The anti-bedsore bed according to the present invention can change the posture of a care-receiver A in the bedridden state, similarly to the physical movement of healthy people, at a particular interval t and thus removes muscle stiffness and prevents development of bedsore of the care-receiver.
It is also possible to remove the muscle stiffness of care-receiver in the regions, by placing the blood circulation-accelerating units in the possible bedsore regions.

### BRIEF DESCRIPTION OF THE DRAWINGS

Figure 1 is a perspective view illustrating an anti-bedsore bed in an embodiment of the present invention.
Figure 2 is a top view of the bed.
Figure 3 is a crosssectional side view of a main part of the bed.
Figure 4 is a perspective view illustrating air bags and the regions where the air bags are connected to.
Figure 5 is an explanatory drawing illustrating the display unit of a control means.
Figure 6 is a crosssectional side view of main parts for explaining the operation of the air bags.
Figure 7 is a crosssectional side view of main parts for explaining the operation of the air bags.
Figure 8 is a crosssectional side view of main parts for explaining the operation of the air bags.
Figure 9 is a crosssectional side view of main parts for explaining the operation of the air bags.

### EXPLANATION OF REFERENCES

1, 1a, 1b, 1c, 1d, and 1e: air bags
2: Air-supplying/discharging means
3: Control means
4: Blood circulation-accelerating unit
10: Bag main body
A: Care-receiver
a: Sural region
b: Femoral region
c: Lumbar region
d: Back region
e: Suboccipital region
t: Particular interval

### BEST MODE OF CARRYING OUT THE INVENTION

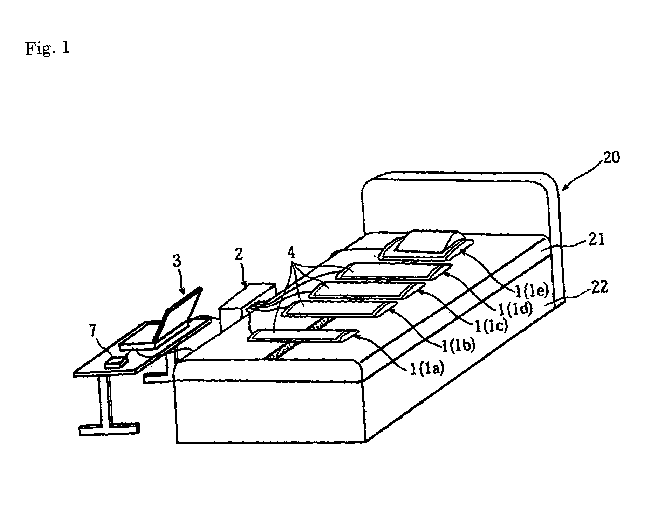

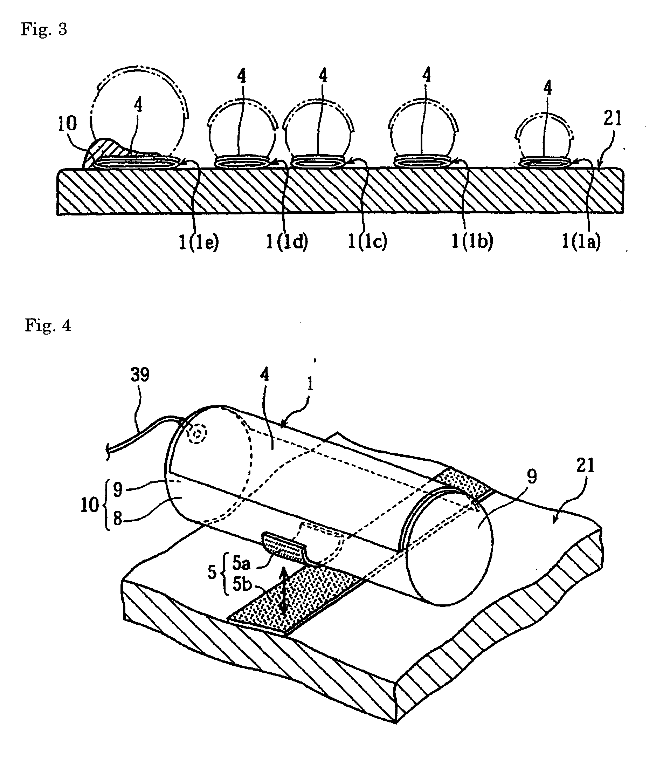

Hereinafter, the present invention will be described in detail with reference to favorable embodiments shown in drawings.
An anti-bedsore bed in an embodiment of the present invention is shown in Figures 1 to 4. The bed according to the present invention is mainly used for prevention of bedsore of care-receivers A such as serious patients and elderly in the physically disabled and bedridden state, as it is installed, for example, in a general hospital or a nursing home for the elderly.
The bed has a bed main body 20 having a mattress 21 and a frame 22, and multiple expandable/contractable air bags 1 placed in parallel with each other in the width direction of the bed, an air-supplying/discharging means 2 of expanding and contracting the air bags 1 by supplying and discharging compressed air into and out of the air bags 1 at an particular interval t, and a control means 3 of controlling the air-supplying/discharging means 2.

Each air bag 1 is formed cylindrical in its expansion state, and used mainly for prevention of bedsore, as it is used for elevating and lowering the region of possible bedsore of the care-receiver A at a particular interval t.
Each air bag 1 has a blood circulation-accelerating unit 4 made of a material accelerating blood circulation of the care-receiver A. Specifically, the air bag 1 has a bag main body cylindrical in the expanded state 10 and a flexible sheet-shaped blood circulation-accelerating unit 4 placed on the bag main body 10.
The bag main body 10 is made of a easily deformable material such as rubber and has a cylindrical region 8 and terminal wall regions 9 closing the opening of the cylindrical region 8 at both sides. The terminal of an air pressure line 39 connected to the air-supplying/discharging means 2 is connected to the terminal wall region 9 of each bag main body 10.

The blood circulation-accelerating unit 4 is prepared by blending and molding a mixture of a powder such as of magnet, tourmaline, germanium, Togoal Warmtite, quartz diorite porphyrite, granite porphyry, Hornblende-cummingtonite, Chinese silica, silicon compound, lead-containing barite, or Bincho charcoal, and a synthetic resin or rubber. The synthetic resin is, for example, a nylon resin.
The Togoal Warmtite is an easily-soluble weathered ore commonly called "Togoal ore". The quartz diorite porphyrite is a sea-bottom sedimentary ore commonly called "Ioseki". The granite porphyry is a composite ore of quartz and feldspar normally called "Bakuhanseki". The Hornblende-cummingtonite is an ore called Aura stone. The Chinese silica is an ore occasionally called black silica or white silica. The lead-containing barite is an ore called hokutolite.
Togoal Warmtite, quartz diorite porphyrite, granite porphyry, Hornblende-cummingtonite, Chinese silica, silicon compound, and lead-containing barite contain favorable minerals advantageous to the body. These ores and also Bincho charcoal have a blood circulation-accelerating action for example by emission of far-infrared ray and ions.

The mechanism of the action of the magnetic force from magnet on muscle stiffness will be described below.
Most stiffness is ischemic muscle pains due to decrease in blood circulation caused by constriction of blood vessels under pressure.
Blood vessels have a property to contract consistently, but blood vessels repeat expansion and contraction at a certain rhythm, as a blood vessel-dilating substance called acetylcholine is released regularly from nerve terminals. When applied to the stiffened region, the magnetic force has an action to delay decomposition of the acetylcholine. Temporal increase in concentration of the vasodilating substance acetylcholine in blood vessel leads to significant dilation of the blood vessel, which in turn leads to acceleration of blood circulation and thorough removal of residual waste products such as lactic acid by flushing. The action of magnetic force on muscle stiffness is based on the blood circulation-accelerating action described above.

Alternatively, tourmaline generates negative ions under external pressure. The negative ions are effective in relaxing the care-receiver A mentally and physically and also in improving physical predisposition and immunological activity. In addition, tourmaline accelerates blood circulation of the care-receiver A by emitting far-infrared ray.
Alternatively, germanium generates negative ions and, by action of the negative ions, accelerates blood circulation of the care-receiver A and alleviates stiffness and fatigue of muscle.

Each air bag 1 is fixed detachably to the top face of a mattress 21 (for example) made of a low-repulsion material. Specifically, each air bag 1 is fixed to the mattress 21 with a sheet-shaped hook and loop fastener 5. A fastening region 5a of the sheet-shaped hook and loop fastener 5 is bonded to the air bag 1 (more specifically, bag main body 10), while the fastened region 5b of the sheet-shaped hook and loop fastener 5 is bonded to the mattress 21. The fastened region 5b of the sheet-shaped hook and loop fastener 5 is formed on the mattress 21 crosswise in the longitudinal direction, thus allowing placement of each air bag 1 at any position of the mattress 21 in the longitudinal direction.

In the present embodiment, the air bags 1 are placed at five positions respectively corresponding to the sural region a, femoral region b, lumbar region c, back region d, and suboccipital region e of the care-receiver A in the supine position (each of the air bags 1 is referred to as 1a, 1b, 1c, 1d, or 1e). The air bag 1e corresponding to the suboccipital region e is also used for bringing the upper half body of the care-receiver A in the upright position, while the air bags 1b and 1c corresponding to femoral region b and lumbar region c are used also for lifting the lumbar region during exchange of the care-receiver A's diaper.
Among the multiple air bags 1, the air bag 1 corresponding to the suboccipital region e has a larger diameter (e.g., diameter of 50 cm), the air bags 1 corresponding to femoral region b, lumbar region c and back region d, a medium diameter (e.g., diameter of 40 cm); and the air bag 1 corresponding to the sural region a has a smaller diameter (e.g., diameter of 30 cm). Generally, the regions of possible bedsore of the care-receiver A (high-incident bedsore region) include heel regions f, pelvic region g, scapular region h, and supraoccipital region i. In particular, the first bedsore-developing region is considered to be the heel regions f. In the present embodiment, the air bags 1 placed at positions corresponding to the sural region a, femoral region b, lumbar region c, and suboccipital region e of the care-receiver A are designed to elevate and lower the heel regions f, pelvic region g, scapular region h, and supraoccipital region i of the care-receiver A at a particular interval t.

The air-supplying/discharging means 2 is installed as an independent unit placed by the bed main body 20. The air-supplying/discharging means 2 has, for example, an air compressor 35, an air tank 36, a regulator 37, and a solenoid valve 38. The air-supplying/discharging means 2 and each air bag 1 are connected to each other with the air pressure line 39 described above. The control means 3 is a so-called personal computer installed by the bed main body 20 (the control means 3 will be described below in detail).

As described above, the air bags 1 are used mainly for prevention of bedsore, but, in the bed in the present embodiment, the air bag 1e corresponding to the suboccipital region e is used for bringing the upper half body of the care-receiver A in the upright position, while the air bags 1b and 1c corresponding to the femoral region b and lumbar region c are used in operation to raise the lumbar region during exchange of the care-receiver A's diaper.

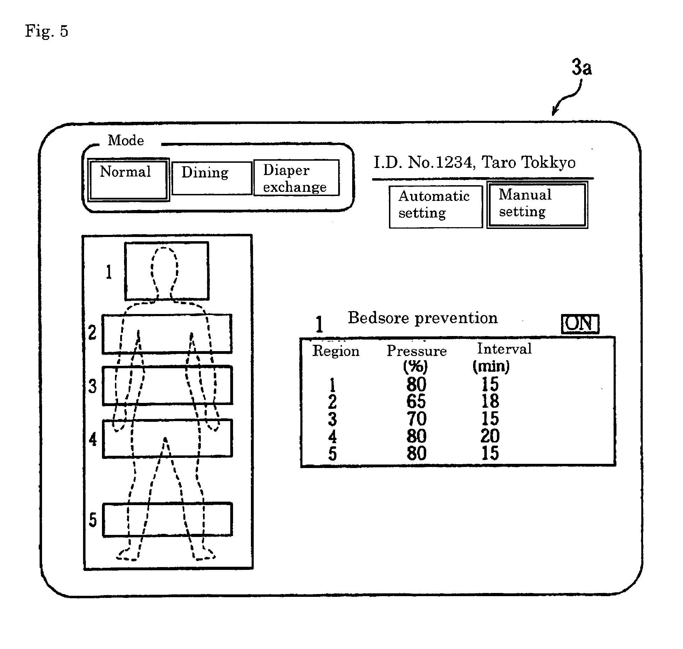

Hereinafter, the control means 3 installed by the bed main body 20 will be described in detail.
The control means 3 is operated by a qualified person B, such as professional instructor, masseur, or physician, who gives guidance on rehabilitation of the care-receiver A and manages treatment. The control means 3 is electrically connected to the air-supplying/discharging means 2 supplying air into the air bags 1 and controls the air-supplying/discharging means 2.
More specifically, the air-supplying/discharging means 2 controls, for example, selection of the air bag 1 to be operated, the intensity of air pressure (e.g., very weak, weak, intermediate, or strong), and the particular interval t (interval of the compressed air to be sent to the air bags 1). In the present embodiment, the particular interval t is set to a value in the range of 15 to 30 minutes. It is possible in this way to change the posture of the care-receiver A properly according to the principle of human physical movement and thus prevent development of bedsore effectively.
As shown in Figure 5, the qualified person B confirms the operation and settings of the air-supplying/discharging means 2 in the display unit 3a of control means 3.

The bed in the present embodiment has a recording medium 6 storing the treatment data of each care-receiver A previously inputted by a qualified person B and a data-reading unit 7 reading the treatment data on the recording medium 6 as it is brought closer to or inserted in the unit.
The treatment data include the setting condition for the air-supplying/discharging means 2 described above and also patient information. The patient information include identification number, name, age, sex, date of birth, blood type, occupation, address, phone number, and current suffering disease.
Examples of the recording media 6 include IC memory chip, IC memory card, magnetic card, magneto-optical card and the like.
The data-reading unit 7 is electrically connected to the control means 3, so that the treatment data on the recording medium 6 can be sent to the control means 3. The control means 3 control operation of the air bags 1 (via the air-supplying/discharging means 2) according to the treatment data in the recording medium 6 which are read by the data-reading unit 7. It is possible in this way to obtain a bed suitable for the taste of care-receivers as a medical device and allowing operation of the air bags under a condition suitable from the profession point of view and to prevent development of bedsore in care-receivers reliably.

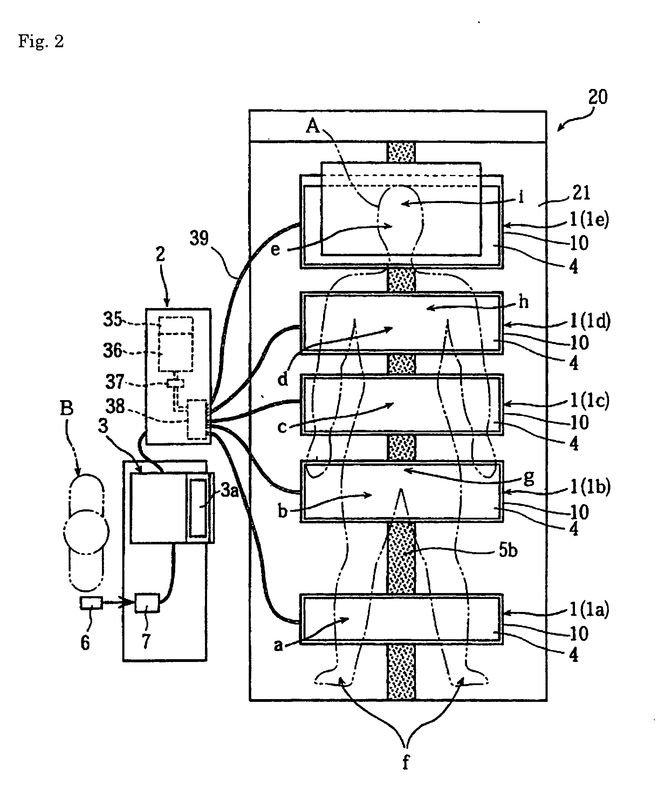

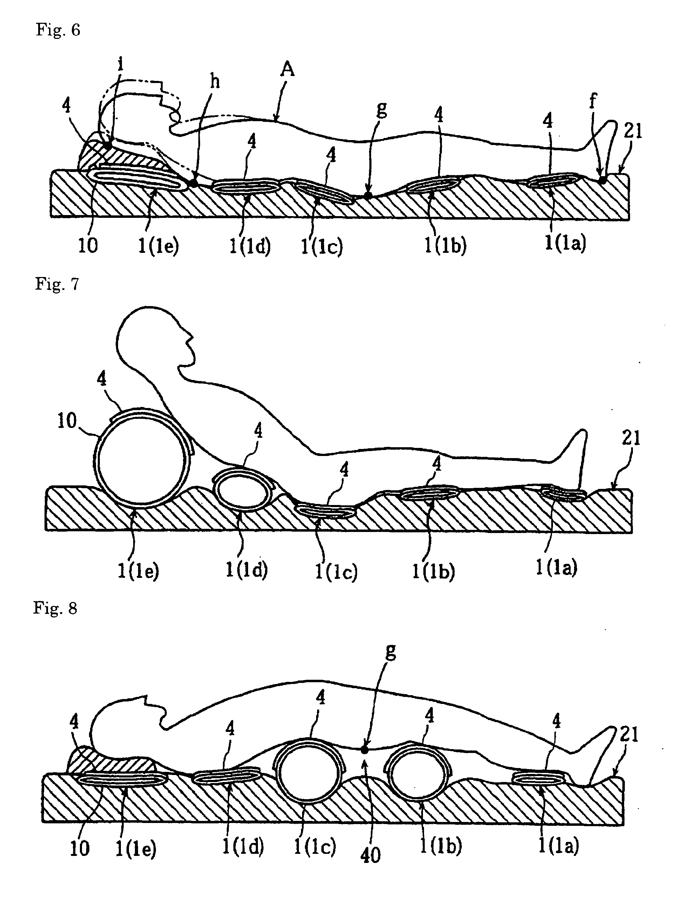

Hereinafter, operation of the bed in the present embodiment will be described with reference to Figures 2 and 6 to 9.
First, as shown in Figures 2 and 6, after a care-receiver A is placed in the supine position on a mattress 21, air bags 1 are placed at positions adjusted to correspond to five positions, the sural region a, femoral region b, lumbar region c, back region d, and suboccipital region e of the care-receiver A. The positioning can be done easily, because each air bag 1 is connected to the mattress 21 with a sheet-shaped hook and loop fastener 5. Specifically, it is possible to adjust the positions thereof according to the individual care-receiver A different in figure and stature. The air bags 1, which are made of an easily deformable material, deform along the figure of the care-receiver A when it is not expanded with air. In this way, the air bags do not pressurize the care-receiver A strongly locally.
In the state, the blood circulation-accelerating unit 4 formed on each air bag 1 accelerates blood circulation in the sural region a, femoral region b, lumbar region c, back region d, and suboccipital region e of the care-receiver A, allowing mental and physical relaxation and removal of muscle stiffness of the care-receiver A.

Then, a data-reading unit 7 connected to a control means 3 reads the treatment data (previously recoded) of the care-receiver A from a recording medium 6 (previously recorded).
A condition suitable for the care-receiver A is set, based on the treatment data.
Air is supplied to each air bag 1 according to the condition suitable for the care-receiver A, and the possible bedsore regions of the care-receiver A (heel regions f, pelvic region g, scapular region h, and supraoccipital region i) are elevated and lowered at a particular interval t. When air is supplied to each air bag 1 for prevention of bedsore, the air bag 1 is not expanded to the maximum, but only slightly to a degree that the body of the care-receiver A is lifted gently (as indicated by the chain double-dashed line in Figure 6). In this way, no large load is applied to the body of the care-receiver A by operation of the air bags 1.

Then as shown in Figures 2 and 7, when the care-receiver A takes a meal, watch television programs, or read a book, the care-receiver changes its operation mode with the control means 3, expanding only the air bag 1d and 1e corresponding to the back region d and the suboccipital region e. In particular, the air bag 1e corresponding to the suboccipital region e is expanded larger. Because the air bag 1e corresponding to the suboccipital region e is formed larger than any other air bags 1, the upper half of the body of the care-receiver A is raised significantly. Then, the care-receiver A can sit up easily without need for any force applied to the abdominal and back muscles. Thus, the care-receiver can take a meal, watch TV programs or read a book in the comfortable position. In this way, the air bag 1 corresponding to the suboccipital region e is used also for the purpose of raising the upper half of the body of the care-receiver A. The entire system is similar in structure, because there is no need for a motor or a link mechanism for raising the body of the care-receiver.

Then as shown in Figures 2 and 8, when the diaper of the care-receiver A is exchanged, the operational mode is altered with the control means 3 for expansion only of the air bags 1b and 1c corresponding to the femoral and lumbar regions of b and c. The lumbar region (pelvic region g) of the care-receiver A is lifted higher, forming a large space 40 between the air bag 1b corresponding to the femoral region b and the air bag 1c corresponding to the lumbar region c. The diaper of the care-receiver A is exchange, manually through the space 40. In this way, the air bags 1 corresponding to the femoral region b and the lumbar region c are used also for the purpose of lifting the lumbar region of the care-receiver A during diaper exchange.

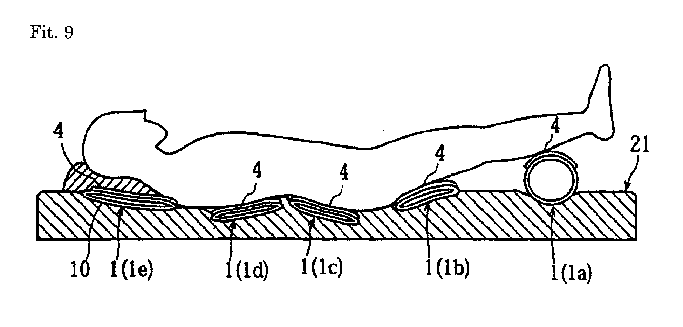

Although only the air bags 1d and 1e corresponding to the back region d and the suboccipital region e are expanded significantly when the care-receiver A takes a meal, watches TV programs or reads a book and only air bags 1b and 1c corresponding to the femoral region b and the lumbar region c during exchange of care-receiver A diaper, the heel regions f, incipient region of bedsore in the care-receiver A, can be raised higher, if the air bag 1a corresponding to the sural region a is expanded significantly, as shown in Figure 9.

The present invention is not limited to the embodiments described above and can be modified in design arbitrarily within the technical scope of the present invention. Although the position of the air bags 1 correspond to the sural region a, femoral region b, lumbar region c, back region d, and suboccipital region e of the care-receiver A in the supine position are exemplified in the present embodiment, the present invention is not limited thereto, and the position of the air bags 1 may favorably correspond to a combination of two or more positions among them.

Although the bed in the present embodiment does not have a massaging function, the present invention is not limited thereto, and a massage function may be added for prevention of bedsore of the care-receiver A and also for relaxation of the care-receiver A by massage. In the configuration above, it is possible to relax care-receivers A integrally for example with heat, massaging and vibration and thus to reduce the stress applied to the care-receiver A more effectively.

As described above, the anti-bedsore bed according to the present invention is an anti-bedsore bed comprising multiple expandable/contractable air bags 1 placed in parallel with each other in the width direction of the bed, an air-supplying/discharging means 2 of expanding and contracting the air bags 1 by supplying and discharging compressed air into and out of the air bags 1 at a particular interval t, and a control means 3 of controlling the air-supplying/discharging means 2, wherein the air bags 1 are formed cylindrical in the expanded state in such a manner that possible bedsore-developing regions of the care-receiver A are elevated and lowered at a particular interval t, and the air bags 1 has a blood circulation-accelerating unit 4 made of a material accelerating blood circulation of the care-receiver A, and thus can change the position of a care-receiver A in the bedridden state, similarly to the physical movement of healthy people, at a particular interval t, and thus to prevent generation of bedsore. The blood circulation-accelerating units 4 places in the possible bedsore regions, and which accelerates of the blood circulation of the care-receiver A in the regions, consequently removing muscle stiffness of the care-receiver A.

Because acceleration of the blood circulation of the care-receiver A by the blood circulation-accelerating unit 4 is preserved, independently of whether the air bags 1 are expanded or contracted by the air-supplying/discharging means 2. Thus, compared to when no blood circulation-accelerating unit 4 is installed, it is possible to reduce the period of expanding and contacting the air bags 1 and thus allow conservation of electricity and fuel.
In addition, air bags 1 formed cylindrical in the expanded state are simpler in shape than air bags 1 in the spherical or striped shape, and are thus produced cost-effectively.

The blood circulation-accelerating unit 4, which is formed by molding a blend of a powder of magnet, tourmaline, germanium, Togoal Warmtite, quartz diorite porphyrite, granite porphyry, Hornblende-cummingtonite, Chinese silica, silicon compound, lead-containing barite, or Bincho charcoal with a synthetic resin or rubber, is flexible, allows easily expansion-contraction of the air bags 1, and accelerates blood circulation by emitting magnetic force and negative ions. It is thus possible to obtain the favorable effects of reducing the muscle stiffness and preventing bedsore of the care-receiver A more efficiently.

Because each air bag 1 has a bag main body cylindrical in the expanded state 10 and a flexible sheet-shaped blood circulation-accelerating unit 4 formed on top of the bag main body 10, a blood circulation-accelerating unit 4 can be formed only in the region in contact with the care-receiver A both in the contracted and expanded states in the air bags 1 above. It is thus possible to form a bag main body 10 with a cheap material and reduce the area of the blood circulation-accelerating unit 4, which is made of a material more expensive than the material of the bag main body 10 and yet obtain an air bag 1 more effective in removing muscle stiffness.

In addition, because the air bags 1 are located at positions corresponding to the sural region a, femoral region b, lumbar region c, back region d, and suboccipital region e of the care-receiver A in the supine position, the bed is effective in removing muscle stiffness and preventing bedsore development over the wide range of possible bedsore regions of the care-receiver A.

### Industrial Applicability

The anti-bedsore bed according to the present invention can be used for prevention of bedsore development in care-receivers such as bedridden patients by disease and bedridden elderly effectively without labor, and thus, will be significantly effective for reduction of the labor of the care-givers and optimization of nursing-care of the care-receivers.

## Claims

1. An anti-bedsore bed, comprising
multiple expandable/contractable air bags (1) placed in parallel with each other in the width direction of the bed, an air-supplying/discharging means (2) of expanding and contracting the air bags (1) by supplying and discharging compressed air into and out of the air bags (1) at a particular interval (t), and a control means (3) of controlling the air-supplying/discharging means (2), wherein
the air bags (1) are formed cylindrical in the expanded state in such a manner that possible bedsore-developing regions of a care-receiver (A) are elevated and lowered at a particular interval (t), and
the air bags (1) has a blood circulation-accelerating unit (4) made of a material accelerating blood circulation of the care-receiver (A).

2. The anti-bedsore bed according to Claim 1, wherein the blood circulation-accelerating unit (4) is formed by molding a blend of a powder of magnet, tourmaline, germanium, Togoal Warmtite, quartz diorite porphyrite, granite porphyry, Hornblende-cummingtonite, Chinese silica, silicon compound, lead-containing barite, or Bincho charcoal with a synthetic resin or rubber.

3. The anti-bedsore bed according to Claim 1 or 2, wherein the air bag (1) has a bag main body (10) formed cylindrical in the expanded state and a flexible sheet-shaped blood circulation-accelerating unit (4) formed on top of the bag main body (10).

4. The anti-bedsore bed according to any one of Claims 1 to 3, wherein the air bags (1) are arranged at positions corresponding to at least two positions of sural region (a), femoral region (b), lumbar region (c), back region (d), and suboccipital region (e) of the care-receiver (A) in the supine position.

5. The anti-bedsore bed according to Claim 4, wherein the air bags (1) are arranged at the positions corresponding to the sural region (a), femoral region (b), lumbar region (c), back region (d), and suboccipital region (e) of the care-receiver (A) in the supine position
